Europäisches Patentamt

European Patent Office

Office européen des brevets

(11) Publication number: **0 345 081**
**A2**

(12) **EUROPEAN PATENT APPLICATION**

(21) Application number: 89305582.2

(22) Date of filing: 02.06.89

(51) Int. Cl.⁴: **A 61 K 7/42**
**A 61 K 31/19**

(30) Priority: 02.06.88 JP 134482/88
21.01.89 JP 12223/89

(43) Date of publication of application:
06.12.89 Bulletin 89/49

(84) Designated Contracting States: DE FR GB

(71) Applicant: KABUSHIKI KAISHA HAYASHIBARA
SEIBUTSU KAGAKU KENKYUJO
2-3, 1-chome, Shimoishii
Okayama-shi Okayama (JP)

Sansho Seiyaku Co., Ltd.
26-7, Oike 2-chome
Onojo-shi Fukuoka-ken (JP)

Mishima, Yutaka
4-32, 1-chome, Sowa-cho
Nada-Ku Kobe-shi Hyogo (JP)

(72) Inventor: Mishima, Yutaka
4-32, 1-chome, Sowa-cho
Nada-ku Kobe-shi Hyogo (JP)

Oyama, Yasuaki
15-16, 2-chome, Oike
Onojo-shi Fukuoka (JP)

Kurimoto, Masashi
28-5, 3-chome, Ifuku-cho
Okayama-shi Okayama (JP)

(74) Representative: Pendlebury, Anthony et al
PAGE, WHITE & FARRER 54 Doughty Street
London WC1N 2LS (GB)

(54) Enzyme formation suppressing agent.

(57) The present invention relates to a pharmaceutical agent to suppress the formation of an enzyme, more particularly, to an entirely novel pharmaceutical agent which does not inhibit the action or activity of tyrosinase which has been formed, but prevents its formation. The pharmaceutical agent comprises one or more members selected from the group consisting of propionic acid, butyric acid and valeric acid, and their salts and derivatives as the effective component.

EP 0 345 081 A2

## Description

## ENZYME FORMATION SUPPRESSING AGENT

### Background of the Invention

#### 1. Field of the invention

The present invention relates to a pharmaceutical agent to suppress the formation of an enzyme, more particularly, to an entirely novel pharmaceutical agent which does not inhibit the action or activity of tyrosinase which has been formed, but prevents its formation.

#### 2. Description of the prior art

The melanin, present in the deeper part of the skin, plays an important role in protecting human body from the affects of ultraviolet rays, as well as being one of important factors in medical science and cosmetology. It has been known that tyrosinase catalyzes the formation of melanin while converting tyrosine, for example, successively into dopa, dopaquinone and 5,6-dioxine indol in a pigment cell of the skin tissue. The presence of an excessive melanin darkens the skin, and its unhomogeneous distribution causes chloasma and freckle. Therefore, melanin is unnegligible in cosmetology.

Conventionally, hydroquinone and MBEH (monobezyl ether of hydroquinone) have been used to realize a pigmentation-lightened skin because of their strong pigmentation-lightening effect, but have the following drawbacks that their administration result in the denaturation and death of pigment cells, that they damage the physiology which is inherent to the skin, and that they may cause undesirable side effects, for example, irreversible alphos, pigmentary disorder and contact dermatitis.

Vitamin C, glutathione and cysteine have been used to reduce the level of melanin in the skin, as well as to realize a pigmentation-lightened skin with the viewpoint that tyrosinase is responsible for the formation of melanin (as disclosed in Japanese Patent Laid-Open No. 142,515/78).

However, pharmaceutical agents such as vitamin C, glutathione and cysteine inhibit the action or activity of formed tyrosinase. As mentioned in the above, the technical level in the prior art lies in how to inhibit the activity of formed tyrosinase.

While the present invention prevents the formation of tyrosinase per se, and does not relate to the treatment of tyrosinase as in conventional procedure. In other words, the present invention relates to the most essential part in the melanogenic system wherein the formation of tyrosinase, which is an essential cause of dermatological trouble, is prevented. Since the technical conception of the present invention is essentially different from that of the conventional procedure, and the original object of the present invention is different even in objects from that of the conventional procedure, and the starting point of the present invention has not been recognized heretofore.

As evident from the above, the present invention is novel with respect to technical subject or object because the technical conception of the present invention has not been recognized, and the specific organic acids, such as propionic acid, butyric acid, valeric acid, and their salts and derivatives, are employed in combination with or without unsaturated fatty acid(s) in the present invention. Additionally, since neither disclosure nor suggestion of the present invention has been shown in the prior art, the present invention is novel.

The effects of the present invention is novel and superior because the object and arrangement of the invention are novel.

Conventional tyrosinase inhibitors are insufficient in inhibitory activity on tyrosinase and also in pigmentation-lightening effect on viable cells because such inhibitors are reducing agents utilizing their inherent reducing power, and because their stability and durability are poor (as disclosed in Japanese Patent Laid-Open No. 142,515/78, page 1, right lower column through page 2, left upper column).

### Summary of the Invention

The present invention is to overcome these drawbacks of conventional method. In conventional method, substances to inhibit the activity of tyrosinase have been used, and these inhibitory substances have proved uneffective in their tyrosinase inhibitory activity and their durability.

Many substances have been extensively tested and studied on their possible most aspects, but it was confirmed that no inhibitory substance completely accomplished a desired effect if tyrosinase is once formed.

The present inventors completely changed the way of approach and investigated pharmaceutical agents capable of preventing tyrosinase formation with the viewpoint that dermatological trouble never occur if no tyrosinase is formed.

In order to accomplish the object, the present inventors investigated substances to suppress the formation of tyrosinase (hereinafter referred to as "tyrosinase formation suppressing substance") while studying organic acids and fatty acids with a high-stability which were responsible for metabolism in order to obtain a substance which suppressed tyrosinase formation via the self-controlling function of a viable pigment cell and exhibited melanogenesis suppressing action independent on the direct inhibition of tyrosinase.

As a result, the present inventors found that propionic acid, butyric acid, valeric acid, and their salts and derivatives in combination with or without unsaturated fatty acid(s) strongly suppressed the formation of tyrosinase, and confirmed that such organic acids were suitable for tyrosinase formation suppressing agent. Furthermore, the present inventors found that a tyrosinase formation suppressing agent containing the substance(s) exerted a melanogenesis suppressing effect, and attained a satisfiable pigmentation-lightening effect. This is the present invention.

Detailed Description of the Invention

The present invention relates to a pharmaceutical agent to suppress the formation of an enzyme, more particularly, to an entirely novel pharmaceutical agent which does not inhibit the action or activity of tyrosinase which has been formed, but prevents its formation.

The acetic acid, lactic acid, pyruvic acid, propionic acid, butyric acid, valeric acid, and their salts and derivatives which are used in the present invention include their isomers, alkali metal salts and alkaline-earth metal salts, and their esters and acid amides.

The unsaturated fatty acids used in the present invention are those having 12 to 22 carbon atoms, for example, lauroleic acid (12:1), myristoleic acid (14:1), pentadecenoic acid (15:1), cis-9-hexadecenoic acid (16:1), oleic acid (18:1), linoleic acid (18:2), linolenic acid (18:3), gadoleic acid (20:1), arachidonic acid (20:4), 4,8,12,16-eicosatetraenoic acid (20:4), 5,8,11,14,17-eicosapentaenoic acid (20:5), erucic acid. (22:1), clupanodonic acid (22:5), and their alkali metal salts and alkaline-earth metal salts, and their esters and acid amides.

Additionally, the tyrosinase formation suppressing agent according to the present invention is characterized in that it substantially does not inhibit tyrosinase activity, but has a superior tyrosinase suppressing action on a viable cell.

Methods to detect several activities described in the specification are as follows:

(1) The tyrosinase activity is detected in accordance with the method described in British Journal of Dermatology, Vol. 103, pp. 625-633 (1980). In the method, a test sample is allowed to react at a prescribed pH. Tyrosinase inhibitory activity is judged positive when the test sample lowers the activity of tyrosinase as compared with a control.

(2) The melanogenesis suppressing action in a prescribed pigment cell is detected in accordance with the method described in Cancer Research, Vol. 42, pp. 1994-2002 (1982).

B-16 cell derived from mouse melanoma is suspended in 10 ml Eagle's minimal essential medium (Grand Island Biological Co., Grand Island, N. Y.), supplemented with 10 v/v % fetal calf serum, to give $4 \times 10^4$ cells. The cell suspension is placed in a 25 cm² Roux's flask, and cultured at 37°C with 5 v/v % $CO_2$. The cultivation is continued for 5 days while replacing the culture medium with fresh culture medium containing a tyrosinase formation suppressing agent on the first- and third-days. After completion of the cultivation, the resultant cells are washed with phosphate buffer (pH 7.2) containing 0.8 w/v % saline, detached from the inside wall of the Roux's flask by the addition of a solution containing trypsin and EDTA (ethylenediaminetetraacetic acid), and collected by filtration method. The cells collected on a filter paper are measured for their reflection light at a wave length of 500 nm with a densitometer, followed by determination of the total amount of melanin in the cells with the reflection absorbance (degree of darkness).

The specimen is judged positive when the reflection absorbance of B-16 cells treated with the specimen is decreased to 80% or lower of the reflection absorbance of B-16 cells without treatment of a tyrosinase formation suppressing agent.

(3) Tyrosinase formation suppressing activity is detected in accordance with the method of dopa-reaction described in Japanese Journal of Dermatology, Vol. 87, No. 13, pp. 883-901 (1977), and detected in accordance with the method of tyrosinase described in Cancer Research, Vol. 42, pp. 1994-2002 (1982). B-16 cell derived from mouse melanoma is cultured in the presence of a tyrosinase formation suppressing agent for 5 days in accordance with the above methods. After the cell is whitened, the culture medium is removed and the cell is fixed in 10 v/v % formaldehyde solution. The cell is then soaked in 0.1 M phosphate buffer (pH 7.3) containing 0.1 w/v % L-dopa, followed by 3-hour standing at 37°C. Tyrosinase formation suppressing activity is detected by the fact that the cell is not darkened when observed microscopically, and by the fact that the reduction or disappearance of tyrosinase isoenzymes $T_1$, $T_2$ and $T_3$ is electrophoretically observed.

Since acetic acid, lactic acid, pyruvic acid, propionic acid, butyric acid, valeric acid, and their salts and derivatives usable in the present invention as tyrosinase formation suppressing substances are very safe and stable, they can be used in combination with unsaturated fatty acid(s) as the effective component in pharmaceutical agents, for example, injection, internal medicine, medicine for external application, and bath preparations such as bath salts and bath liquids; and in cosmetics, for example, milky lotion, pack, and cream. Furthermore, the tyrosinase formation suppressing substances exert a high prophylactic and therapeutic effect on chromatosis, for example, chloasma, freckle, and sunburn.

The specific organic acids, and their salts and derivatives, and unsaturated fatty acids in the present invention can be used alone or in combination, and the contribution results in a synergistic effect.

As apparent from the fact that we take these organic acids, and their salts and derivatives which are contained in some foods though their amounts are small, as well as being derived from enterobacteria, and that unsaturated fatty acids are the components of the body and natural substances, as well as being orally administrable, such organic acids, and their salts and derivatives do not have a substantial toxicity, and they are usable with no side effect.

The present invention will hereinafter be explained in more detail with the following Experiments.

Experiment-1

Study of tyrosinase formation suppressing substance

Experiment 1-(1)

Organic acids, and their salts and derivatives

According to the methods described in the above, organic acids as shown in Table 1 which were responsible for metabolism were studied their tyrosinase inhibitory activity, melanogenesis suppressing action and tyrosinase formation suppressing action. Each organic acid was previously neutralized with sodium hydroxide to a predetermined pH, and used at a concentration of 10 mM.

The results are as shown in Table 1.

Table 1

| | Tyrosinase inhibitory action | Melanogenesis suppressing action | Tyrosinase formation suppressing action | Remarks |
|---|---|---|---|---|
| Propionic acid | - | + | + | Present invention |
| Normal butyric acid | - | + | + | Present invention |
| Normal valeric acid | - | + | + | Present invention |
| Aspartic acid | - | - | - | Control |
| Glutamic acid | - | - | - | Control |
| Citric acid | - | - | - | Control |
| Fumalic acid | - | - | - | Control |
| Malic acid | - | - | - | Control |
| Vitamin C | + | - | - | Control |

(Note 1)  When organic acids had optical isomers, their L-configurations were used.
(Note 2)  The symbol "+" means that inhibiting- or suppressing-action is detected, and the symbol "-" means that no inhibiting- or suppressing-action is detected.

As evident from the results in Table 1, it was found that propionic acid, butyric acid and valeric acid unlike other organic acids exhibited tyrosinase formation suppressing action, but not tyrosinase inhibitory action.

Furthermore, it was found that propionic acid, normal butyric acid, isobutyric acid, normal valeric acid and isovaleric acid exhibited a melanogenesis inhibitory action by their tyrosinase formation suppressing action.

Experiment 1-(2)

Fatty acids

Similarly as in Experiment 1-(1), saturated- and unsaturated-fatty-acids as shown in Table 2 were studied their tyrosinase inhibitory activity, melanogenesis suppressing action and tyrosinase formation suppressing action. Each fatty acid was previously neutralized with sodium hydroxide to a predetermined pH, and used at a concentration of 0.2 mM.

The results are as shown in Table 2.

6

Table 2

| | | Tyrosinase inhibitory action | Melanogenesis suppressing action | Tyrosinase formation suppressing action | Remarks |
|---|---|---|---|---|---|
| Saturated fatty acids | Lauric acid (C12) | - | - | - | Control |
| | Myristic acid (C14) | - | - | - | Control |
| | Palmitic acid (C16) | - | - | - | Control |
| | Stearic acid (C18) | - | - | - | Control |
| | Arachidic acid (C20) | - | - | - | Control |
| | Behenic acid (C22) | - | - | - | Control |
| | Lignoceric acid (C24) | - | - | - | Control |
| Unsaturated fatty acids | Lauroleic acid (12:1) | - | + | + | Present invention |
| | Myristoleic acid (14:1) | - | + | + | Present invention |

EP 0 345 081 A2

(Continued)

| | | Tyrosinase inhibitory action | Melanogenesis suppressing action | Tyrosinase formation suppressing action | Remarks |
|---|---|---|---|---|---|
| Unsaturated fatty acids | Pentadecenoic acid (15:1) | - | + | + | Present invention |
| | Palmitoleic acid (16:1) | - | + | + | Present invention |
| | Oleic acid (18:1) | - | + | + | Present invention |
| | Ricinoleic acid (18:1) | - | + | + | Present invention |
| | Linoleic acid (18:2) | - | + | + | Present invention |
| | Linolenic acid (18:3) | - | + | + | Present invention |
| | Gadoleic acid (20:1) | - | + | + | Present invention |
| | Arachidonic acid (20:4) | - | + | + | Present invention |

EP 0 345 081 A2

(Continued)

| | | Tyrosinase inhibitory action | Melanogenesis suppressing action | Tyrosinase formation suppressing action | Remarks |
|---|---|---|---|---|---|
| Unsaturated fatty acid | 4,8,12,16-eicosatetraenoic acid (20:4) | - | + | + | Present invention |
| | 5,8,11,14,17-eicosapentaenoic acid (20:5) | - | + | + | Present invention |
| | Erucic acid (22:1) | - | + | + | Present invention |
| | Clupanodonic acid (22:5) | - | + | + | Present invention |

(Note)  The symbol "+" means that inhibiting- or suppressing-action is detected, and the symbol "-" means that no inhibiting- or suppressing-action is detected.

EP 0 345 081 A2

As evident from the results in Table 2, it was found that the unsaturated fatty acids unlike saturated fatty acids did not exhibit tyrosinase inhibitory action, but exhibited the formation of tyrosinase.

Furthermore, it was found that the unsaturated fatty acids exhibited melanogenesis suppressing action by their tyrosinase formation suppressing action.

Experiment 2

Effects of salts of organic acids and unsaturated fatty acids, and their concentration on tyrosinase formation suppressing action

Experiment 2-(1)

Organic acids

Similarly as in Experiment 1-(1), effects of salts of propionic acid, butyric acid and valeric acid, and their concentration on tyrosinase formation inhibitory action were studied.

The results are as shown in Table 3.

Table 3

| Concentration (mM) | 1.0 | 2.5 | 5.0 | 10.0 |
|---|---|---|---|---|
| Sodium propionate | - | + | + | + |
| Calcium propionate | - | + | + | + |
| Potassium normal butyrate | - | + | + | + |
| Magnesium isobutyrate | - | + | + | + |
| Lithium normal valerate | - | + | + | + |
| Potassium isovalerate | - | + | + | + |

(Note)    The symbol "+" means that suppressing action is detected,
and the symbol "-" means that no suppressing action is detected.

EP 0 345 081 A2

As evident from the results in Table 3, it was found that similarly as the propionic acid, normal butyric acid, isobutyric acid, normal valeric acid and isovaleric acid, their alkali metal salts, for example, lithium-, sodium- and potassium-salts, and their alkaline-earth metal salts, for example, calcium- and magnesium-salts exhibited a tyrosinase formation suppressing action at a concentration of about 2.5 mM or higher.

Experiment 2-(2)

Unsaturated fatty acids

Similarly as in Experiment 1-(1), effects of salts of pentadecenoic acid (15:1), linolenic acid (18:3) and 4,8,12,16-eicosatetraenoic acid (20:4), and their concentration on tyrosinase formation suppressing action were studied.

The results are as shown in Table 4.

Table 4

| Concentration (mM) | 0.001 | 0.01 | 0.05 | 0.1 | 0.5 |
|---|---|---|---|---|---|
| Sodium pentadecenoate | - | - | + | + | + |
| Potassium pentadecenoate | - | - | + | + | + |
| Potassium linolenate | - | - | + | + | + |
| Lithium linolenate | - | - | + | + | + |
| Sodium eicosatetraenoate | - | - | + | + | + |
| Potassium eicosatetraenoate | - | - | + | + | + |

(Note)    The symbol "+" means that suppressing action is detected,
and the symbol "-" means that no suppressing action is detected.

As evident from the results in Table 4, it was found that the salts of unsaturated fatty acids, for example, those of pentadecenoic acid, linolenic acid and 4,8,12,16-eicosatetraenoic acid, exhibited tyrosinase formation suppressing action at a concentration of about 0.05 mM or higher.

Experiment 3

Augmentation of tyrosinase formation suppressing action

Experiment 3-(1)

Combination of organic acids

Similarly as in Experiment 1-(1), effects of combination of two or more of propionic acid, normal butyric acid and normal valeric acid on tyrosinase formation suppressing action were studied. Each organic acid was previously neutralized with sodium hydroxide to a predetermined pH, and used in a predetermined ratio (molecular ratio) at a predetermined concentration.

The results are as shown in Table 5.

Table 5

| Concentration (mM) | 1.0 | 2.5 | 5.0 | 10.0 |
|---|---|---|---|---|
| Propionic acid | - | + | + | + |
| Normal butyric acid | - | + | + | + |
| Normal valeric acid | - | + | + | + |
| Acetic acid : L-lactic acid <br> 1 : 4 | + | + | + | + |
| Acetic acid : Pyruvic acid <br> 4 : 1 | + | + | + | + |
| L-lactic acid : Pyruvic acid <br> 1 : 1 | + | + | + | + |
| Acetic acid : L-lactic acid : Pyruvic acid <br> 1 : 2 : 1 | + | + | + | + |

(Note)    The symbol "+" means that suppressing action is detected,
and the symbol "-" means that no suppressing action is detected.

EP 0 345 081 A2

As evident from the results in Table 5, it was found that tyrosinase formation suppressing action was more strongly augmented by combination of two or more of propionic acid, normal butyric acid and normal valeric acid than by their sole use.

Experiment 3-(2)

Combination of organic acids and unsaturated fatty acids

Similarly as in Experiment 1-(1), effects of combination of organic acids, for example, propionic acid, normal butyric acid and normal valeric acid, and unsaturated fatty acids, for example, pentadecenoic acid, linolenic acid and 4,8,12,16-eicosatetraenoic acid, on tyrosinase formation suppressing action were studied. The organic acids and unsaturated fatty acids were previously neutralized with sodium hydroxide to a predetermined pH, and used in a predetermined ratio (molecular ratio) at a predetermined concentration.

The results obtained from propionic acid, normal butyric acid and normal valeric acid are as shown in Tables 6-1, 6-2 and 6-2 respectively.

Table  6-1

| | Propionic acid (mM) | 0 | 0.5 | 1.0 | 2.5 | 5.0 |
|---|---|---|---|---|---|---|
| | mM | | | | | |
| Pentadecenoic acid | 0 | − | − | − | + | + |
| | 0.001 | − | + | + | + | + |
| | 0.01 | − | + | + | + | + |
| | 0.05 | + | + | + | + | + |
| | 0.1 | + | + | + | + | + |
| Linolenic acid | 0.001 | − | + | + | + | + |
| | 0.01 | − | + | + | + | + |
| | 0.05 | + | + | + | + | + |
| | 0.1 | + | + | + | + | + |

EP 0 345 081 A2

(Continued)

| | mM | Propionic acid (mM) 0 | 0.5 | 1.0 | 2.5 | 5.0 |
|---|---|---|---|---|---|---|
| | 0.001 | − | + | + | + | + |
| 4,8,12,16-eicosatetraenoic acid | 0.01 | − | + | + | + | + |
| | 0.05 | + | + | + | + | + |
| | 0.1 | + | + | + | + | + |

(Note)  The symbol "+" means that suppressing action is detected, and the symbol "−" means that no suppressing action is detected.

EP 0 345 081 A2

Table 6-2

| | Normal butyric acid (mM) | 0 | 0.5 | 1.0 | 2.5 | 5.0 |
|---|---|---|---|---|---|---|
| | mM | | | | | |
| Pentadecenoic acid | 0 | - | - | - | + | + |
| | 0.001 | - | + | + | + | + |
| | 0.01 | - | + | + | + | + |
| | 0.05 | + | + | + | + | + |
| | 0.1 | + | + | + | + | + |
| Linolenic acid | 0.001 | - | + | + | + | + |
| | 0.01 | - | + | + | + | + |
| | 0.05 | + | + | + | + | + |
| | 0.1 | + | + | + | + | + |
| 4,8,12,16-eicosatetraenoic acid | 0.001 | - | + | + | + | + |
| | 0.01 | - | + | + | + | + |
| | 0.05 | + | + | + | + | + |
| | 0.1 | + | + | + | + | + |

(Note)  The symbol "+" means that suppressing action is detected, and the symbol "-" means that no suppressing action is detected.

Table  6-3

| | Normal valeric acid (mM) | 0 | 0.5 | 1.0 | 2.5 | 5.0 |
|---|---|---|---|---|---|---|
| | mM | | | | | |
| Pentadecenoic acid | 0 | - | - | - | - | + |
| | 0.001 | - | + | + | + | + |
| | 0.01 | - | + | + | + | + |
| | 0.05 | + | + | + | + | + |
| | 0.1 | + | + | + | + | + |
| Linolenic acid | 0.001 | - | + | + | + | + |
| | 0.01 | - | + | + | + | + |
| | 0.05 | + | + | + | + | + |
| | 0.1 | + | + | + | + | + |

EP 0 345 081 A2

(Continued)

| | Normal valeric acid (mM) | 0 | 0.5 | 1.0 | 2.5 | 5.0 |
|---|---|---|---|---|---|---|
| | mM | | | | | |
| 4,8,12,16-eicosatetraenoic acid | 0.001 | − | + | + | + | + |
| | 0.01 | − | + | + | + | + |
| | 0.05 | + | + | + | + | + |
| | 0.1 | + | + | + | + | + |

(Note)     The symbol "+" means that suppressing action is detected, and the symbol "−" means that no suppressing action is detected.

EP 0 345 081 A2

As evident from the results in Tables 6-1, 6-2 and 6-3, it was found that the tyrosinase formation suppressing action was more synergistically augmented by the combination use of organic acids and unsaturated fatty acids than by their sole use.

Experiment 4

Clinical test

Experiment 4-(1)

Organic acids

Tyrosinase formation suppressing agents, prepared by the methods in Examples 4, 6, 8 and 10, were respectively tested for 3 months with 60 male and female volunteers (20 to 55 years old) who had been suffered from chloasma and freckle (number of male and female volunteers were 30 respectively, and their average age were 32.6 and 36.5 respectively). After completion of the test, the volunteers were asked on safeness and efficacy of the agents.

The results are as shown in Table 7.

Table 7

| Examples | 4 | 6 | 8 | 10 |
|---|---|---|---|---|
| Form of tyrosinase formation suppressing agent | Ointment | Lotion | Milky lotion | Cream |
| Chloasma and freckle were turned almost inconspicuous | 20 | 17 | 22 | 28 |
| Color of chloasma and freckle was turned neutral tint | 37 | 35 | 35 | 30 |
| Inefficacious | 3 | 8 | 3 | 2 |
| Side effect was occurred | 0 | 0 | 0 | 0 |

(Note) Values are number of volunteers.

Experiment 4-(2)

Combination of organic acids and unsaturated fatty acids

Tyrosinase formation suppressing agents prepared by the methods in Examples 14, 16, 18 and 20, were respectively tested for 3 months with 60 male and female volunteers (20 to 50 years old) who had been suffered from chloasma and freckle (number of male and female volunteers were 30 respectively, and their average age were 30.6 and 32.5 respectively). After completion of the test, the volunteers were asked on safeness and efficacy of the agents.

The results are as shown in Table 8.

Table 8

| Examples | | 14 | 16 | 18 | 20 |
|---|---|---|---|---|---|
| Form of tyrosinase formation suppressing agent | Ointment | Lotion | Milky lotion | Cream | |
| Chloasma and freckle were turned almost inconspicuous | | 25 | 21 | 24 | 29 |
| Color of chloasma and freckle was turned neutral tint | | 32 | 35 | 34 | 30 |
| Inefficacious | | 3 | 4 | 2 | 1 |
| Side effect was occurred | | 0 | 0 | 0 | 0 |

(Note) Values are number of volunteers.

As evident from the results in Tables 7 and 8, it was found that the present tyrosinase formation suppressing agent was usable with no side effect, and the agent exerted a strong high-efficacy in the prevention and treatment of chloasma and freckle.

As evident from the results in the above Experiments, the above mentioned tyrosinase formation suppressing substances exhibit a tyrosinase formation suppressing action and a strong melanogenesis suppressing effect, and attain a high pigmentation-lightening effect. Furthermore, the tyrosinase formation suppressing substances are extremely safe in view of their effective dose.

The present tyrosinase formation suppressing agent containing as the effective component one or more of acetic acid, lactic acid, pyruvic acid, and their salts and derivatives can be used alone, usually, at a concentration in the range of 0.001-1.0 M, preferably, in the range of 0.005-0.5 M, and at a pH in the range of 2.5-9.0, preferably, in the range of 3.0-8.0. The present tyrosinase formation suppressing agent can be used at a dose of 0.01-100 g/day/adult; preferably, 0.01-10 g/day/adult in systemic administrations such as intramuscular injection; 0.5-100 g/day/adult in oral administrations such as internal medicine; 0.01-100 g/day/adult in percutaneous or permucosal administrations, for example, medicine for external applications, and bath preparations such as bath salts and bath liquids, and cosmetics such as milky lotion and cream. The dose should be changed dependently on the route of administration of the tyrosinase formation suppressing agent and/or patient's symptom.

Furthermore, the present tyrosinase formation suppressing agent containing as the effective component one or more of propionic acid, butyric acid, valeric acid, and their salts and derivatives can be used in combination with unsaturated fatty acid(s), and, usually, such propionic acid, butyric acid, valeric acid, and their salts and derivatives, and unsaturated fatty acids are respectively usable at a concentration in the range of 0.00001-50 w/w %, preferably, in the range of 0.0001-10 w/w %, and at a pH in the range of 2.5-9.0, preferably, in the range of 3.0-8.0. The present tyrosinase formation suppressing agent can be used at a dose of 0.01-500 g/day/adult; preferably, 0.01-50 g/day/adult in systemic administrations such as intramuscular injection; 1.0-100 g/day/adult in oral administrations such as internal medicine; 0.1-500 g/day/adult in percutaneous or permucosal administrations, for example, medicine for external applications, and bath preparations such as bath salts and bath liquids, and cosmetics such as milky lotion and cream. The dose is dependent on the route of administration of the tyrosinase formation suppressing agent and/or patient's symptom.

Such tyrosinase formation suppressing agent containing the tyrosinase formation suppressing substance(s) alone, or, if necessary, in combination with other materials, for example, biologically-active substances, nutrients, bases, and vehicles, can be selected in accordance with the free use and form of pharmaceuticals and cosmetics. The tyrosinase-formation suppressing agent can be used in the prevention or treatment of local chromatosis such as chloasma, freckle and sunburn, as well as of systemic chromatosis such as addisonism.

For example, the tyrosinase formation suppressing agent can be used in the form of injection such as those in liquid or solid which is dissolved in a solvent such as water prior to its use; in the form of oral agents such as those in liquid, powder, granule, capsule, and tablet; in the form of percutaneous agents such as those in liquid, milky lotion, cream and ointment; in the form of medicine for external application such as those in cataplasm which are applied on their one side with an agent such as cream; in the form of bath preparations such as bath salts and bath liquids; and in the form of cosmetics such as lotion, milky lotion, pack, and cream. Since percutaneous administrations augment the tyrosinase formation suppressing action, as well as promoting melanogenesis suppressing effect, penetration of a tyrosinase formation suppressing substance into deeper part of the skin can be promoted by iontophoresis. If necessary, such iontophoresis can be accelerated by the use of a supporting electrolyte.

If necessary, the tyrosinase formation suppressing substance can be freely used in combination with one or more of organic acids, for example, acetic acid, lactic acid and pyruvic acid, and, vitamin C, vitamin E, glutathione, cysteine, kojic acid, placenta extract, glucosamine derivative(s), "PIONIN (KANKOH-SO No. 201)", a product of Nippon Kankoh-Shikiso Kenkyusho, Co., Ltd., Okayama, Japan, colloidal sulfur, and hydroquione derivative(s) in order to promote melanogenesis inhibition and to promote pigmentation-lightening effect. Sunscreen agent can be suitably used in combination with the present agent to promote its efficacy.

Examples of the tyrosinase formation suppressing agent according to the present invention will hereinafter be explained.

### Example 1

#### Injection

Eight parts by weight of sodium propionate, 2.0 parts by weight of L-lactic acid, 10 parts by weight of sodium lactate, 5.3 parts by weight of sodium chloride, 0.3 parts by weight of potassium chloride and 0.2 parts by weight of calcium chloride were dissolved in 1,000 parts by weight of water, and the mixture was membrane-filtered in usual manner. Two milliliter aliquots of the filtrate were distributed into sterilized glass vials and hermetically sealed to obtain an injection.

The injection can be advantageously used as a tyrosinase formation suppressing agent in the prevention and treatment of local chromatosis such as chloasma and freckle, as well as of systemic chromatosis such as addisonism.

## Example 2

Oral agent (tablet)

Forty parts by weight of calcium normal butyrate pentahydrate, 45 parts by weight of maltose, 61 parts by weight of corn starch and 4 parts by weight of sucrose fatty acid ester were homogeneously mixed, and the mixture was tabletted in usual manner to obtain tablets, 150 mg weight each.

Similarly as the product in Example 1, the tablet can be advantageously used as a tyrosinase formation suppressing agent in the prevention and treatment of chromatosis.

## Example 3

Oral agent (capsule)

Ten parts by weight of calcium propionate monohydrate, 50 parts by weight of magnesium L-lactate trihydrate, 87 parts by weight of maltose and 2 parts by weight of sucrose fatty acid ester were homogeneously mixed, and the mixture was fed to a granulator and the resultant was encapsulated in usual manner with gelatin to obtain capsules, 150 mg weight each.

Similarly as the product in Example 1, the capsule can be advantageously used as a tyrosinase formation suppressing agent in the prevention and treatment of chromatosis.

## Example 4

Medicine for external application (ointment)

One part by weight of sodium normal valerate trihydrate and 4.0 parts by weight of calcium isobutyrate were homogeneously mixed with 10.0 parts by weight of glycerine, 50.0 parts by weight of petrolatum, 10.0 parts by weight of vegetable wax, 10.0 parts by weight of lanolin, 14.5 parts by weight of sesame oil, and 0.5 parts by weight of Japanese mint oil to obtain an ointment.

Similarly as the product in Example 2, the ointment can be advantageously used as a tyrosinase formation suppressing agent in the treatment of chromatosis, as well as in the prevention of sunburn.

## Example 5

Medicine for external application (milky lotion)

Two parts by weight of sodium propionate and 3.0 parts by weight of isovaleric acid were homogeneously mixed in usual manner with 12.0 parts by weight of liquid paraffine, 4.0 parts by weight of lanolin, 3.5 parts by weight of oleic acid, 1.0 part by weight of triethanolamine, and 3.0 parts by weight of octyl dodecyl myristate. The mixture was added with small amounts of antiseptic and flavoring agent, and 71.5 parts by weight of purified water, and the resultant was homogenized to obtain a milky lotion.

Similarly as the product in Example 4, the milky lotion can be advantageously used as a tyrosinase formation suppressing agent in the prevention and treatment of chromatosis.

## Example 6

Medicine for external application (lotion)

Two parts by weight of sodium propionate, 1.0 part by weight of hydrogenated castor oil polyoxyethylene, 15.0 parts by weight of ethanol, 0.1 part by weight of DL-lactic acid, 0.3 parts by weight of sodium DL-lactate, 4.0 parts by weight of 1,3-butylene glycol, 0.5 parts by weight of sodium pyrrolidonecarbonate, small amounts of antiseptic and flavoring agent, and 77.0 parts by weight of purified water were homogeneously mixed in usual manner to obtain a lotion.

Similarly as the product in Example 5, the lotion can be favorably used as a tyrosinase formation suppressing agent in the prevention and treatment of chromatosis such as chloasma, freckle and sunburn.

## Example 7

Bath salts

Fifty parts by weight of sodium DL-lactate and 15 parts by weight of sodium propionate were mixed with 35 parts by weight of purified water together with small amounts of coloring- and flavoring-agents to obtain bath salts.

The product can be favorably used as a tyrosinase formation suppressing agent, and can be diluted in 100-10,000 volumes of hot water prior to the use in bath for the prevention and treatment of local and systemic chromatosis. Furthermore, the product exerts a satisfiable melanogenesis suppressing effect even when diluted in solvents such as water, hot water or lotion, prior to use in washing face or bath.

### Example 8

Cosmetic (milky lotion)
A half part by weight of polyoxyethylene behenyl ether, 1.0 part by weight of polyoxyethylene sorbitol tetraoleate, 1.0 part by weight of lipophilic glyceryl monostearate, 0.5 parts by weight of propionic acid, 0.5 parts by weight of behenyl alcohol, 1.0 part by weight of avocado oil, and small amounts of vitamin E, antiseptic and flavoring agent were dissolved by heating in usual manner. To the mixture was added 1.0 part by weight of sodium isobutyrate, 5.0 parts by weight of 1,3-buthylene glycol, 0.1 part by weight of carboxyvinylpolymer, and 85.3 parts by weight of purified water, and the resultant was homogenized to obtain a milky lotion.
The milky lotion can be used as a tyrosinase formation suppressing agent in the prevention and treatment of chromatosis such as chloasma, freckle and sunburn.

### Example 9

Cosmetic (pack)
Two parts by weight of sodium propionate was homogeneously mixed in usual manner with 1.5 parts by weight of squalene, 0.5 parts by weight of hydrogenated castor oil, 4.0 parts by weight of glyceride, 15.0 parts by weight of polyvinyl alcohol, 10.0 parts by weight of ethanol, and 67.0 parts by weight of purified water to obtain a pack.
Similarly as the product in Example 8, the pack can be advantageously used as a tyrosinase formation suppressing agent in the prevention and treatment of chromatosis.

### Example 10

Cosmetic (cream)
Two parts by weight of polyoxyethylene glycol monostearate, 5 parts by weight of glyceryl monostearate SE, 5 parts by weight of propionic acid, 1 part by weight of behenyl alcohol, 1 part by weight of liquid paraffine, 10 parts by weight of glyceryl tri (2-ethylhexanoate), 3 parts by weight of sodium bicarbonate, and small amounts of antiseptic and flavoring agent were dissolved by heating in usual manner. To the mixture was added 2 parts by weight of normal butyric acid, 5 parts by weight of 1,3-buthylene glycol, and 75 parts by weight of purified water, and the resultant mixture was homogenized to obtain a cream.
Similarly as the product in Example 8, the cream can be advantageously used as a tyrosinase formation suppressing agent in the prevention and treatment of chromatosis.

### Example 11

Injection
Eight parts by weight of sodium propionate, 2.0 parts by weight of L-lactic acid, 10 parts by weight of sodium acetate, 5.3 parts by weight of sodium chloride, 0.3 parts by weight of potassium chloride and 0.2 parts by weight of sodium linolenate were dissolved in 1,000 parts by weight of water, and the mixture was membrane-filtered in usual manner. Two milliliter aliquots of the filtrate were distributed into sterilized glass vials and hermetically sealed to obtain an injection.
The injection can be advantageously used as a tyrosinase formation suppressing agent in the prevention and treatment of local chromatosis such as chloasma and freckle, as well as of systemic chromatosis such as addisonism.

### Example 12

Oral agent (tablet)
Forty parts by weight of calcium normal butyrate pentahydrate, 45 parts by weight of maltose, 61 parts by weight of corn starch and 4 parts by weight of sodium linoleate were homogeneously mixed, and the mixture was tabletted in usual manner to obtain tablets, 150 mg weight each.
Similarly as the product in Example 11, the tablet can be advantageously used as a tyrosinase formation suppressing agent in the prevention and treatment of chromatosis.

### Example 13

Oral agent (capsule)
Ten parts by weight of calcium propionate monohydrate, 50 parts by weight of magnesium L-lactate trihydrate, 77 parts by weight of maltose and 10 parts by weight of gamma-cyclodextrin, 2 parts by weight of 5,8,11,14,17-eicosapentaenoic acid were homogeneously mixed, and the mixture was fed to a granulator and the resultant was encapsulated in usual manner with gelatin to obtain capsules, 150 mg weight each.
Similarly as the product in Example 11, the capsule can be advantageously used as a tyrosinase formation lotion can be advantageously used as a tyrosinase formation suppressing agent in the prevention and treatment of chromatosis.

## Example 14

Medicine for external application (ointment)

One part by weight of sodium normal valerate and 4.0 parts by weight of calcium isobutyrate were first homogeneously mixed with 10.0 parts by weight of glycerine, and then the mixture was homogeneously mixed with 50.0 parts by weight of petrolatum, 10.0 parts by weight of vegetable wax, 10.0 parts by weight of lanolin, 14.5 parts by weight of sesame oil, 1.0 part by weight of pentadecenoic acid, and 0.5 parts by weight of Japanese mint oil to obtain an ointment.

Similarly as the product in Example 12, the ointment can be advantageously used as a tyrosinase formation suppressing agent in the treatment of chromatosis, as well as in the prevention of sunburn.

## Example 15

Medicine for external application (milky lotion)

Two parts by weight of sodium propionate and 3.0 parts by weight of isovaleric acid were homogeneously mixed in usual manner with 12.0 parts by weight of liquid paraffine, 4.0 parts by weight of lanolin, 3.5 parts by weight of oleic acid, 1.0 part by weight of triethanolamine, and 3.0 parts by weight of octyl dodecyl myristate. The mixture was added with small amounts of antiseptic and flavoring agent, and 71.5 parts by weight of purified water, and the resultant was homogenized to obtain a milky lotion.

Similarly as the product in Example 14, the milky lotion can be advantageously used as a tyrosinase formation suppressing agent in the prevention and treatment of chromatosis.

## Example 16

Medicine for external application (lotion)

Two parts by weight of sodium propionate, 1.0 part by weight of pentadecenoic acid, 1.0 part by weight of hydrogenated castor oil polyoxyethylene, 15.0 parts by weight of ethanol, 0.1 part by weight of DL-lactic acid, 0.3 parts by weight of sodium normal butyrate, 4.0 parts by weight of 1,3-butylene glycol, 0.5 parts by weight of sodium pyrrolidonecarbonate, small amounts of antiseptic and flavoring agent, and 76.0 parts by weight of purified water were homogeneously mixed in usual manner to obtain a lotion.

Similarly as the product in Example 15, the lotion can be favorably used as a tyrosinase formation suppressing agent in the prevention and treatment of chromatosis such as chloasma, freckle and sunburn.

## Example 17

Bath salts

Twenty-five parts by weight of sodium DL-lactate, 8 parts by weight of sodium propionate, 1 part by weight of oleic acid, and 40 parts by weight of ethanol were mixed with 26 parts by weight of purified water together with small amounts of coloring- and flavoring-agents to obtain bath salts.

The product can be favorably used as a tyrosinase-formation suppressing agent, and can be diluted in 100-10,000 volumes of hot water prior to the use in bath for the prevention and treatment of local and systemic chromatosis. Furthermore, the product exerts a melanogenesis suppressing effect even when diluted in solvents such as water, hot water or lotion, prior to use in washing face or in bath.

## Example 18

Cosmetic (milky lotion)

A half part by weight of polyoxyethylene behenyl ether, 1.0 part by weight of polyoxyethylene sorbitol tetraoleate, 1.0 part by weight of lipophilic glyceryl monostearate, 0.5 parts by weight of propionic acid, 0.5 parts by weight of behenyl alcohol, 1.0 part by weight of avocado oil, 1.0 part by weight of linoleic acid, and small amounts of vitamin E, antiseptic and flavoring agent were dissolved by heating in usual manner. To the mixture was added 1.0 part by weight of sodium isobutyrate, 5.0 parts by weight of 1,3- buthylene glycol, 0.1 part by weight of carboxyvinylpolymer, and 85.3 parts by weight of purified water, and the resultant was homogenized to obtain a milky lotion.

The milky lotion can be used as a tyrosinase formation suppressing agent in the prevention and treatment of chromatosis such as chloasma, freckle and sunburn.

## Example 19

Cosmetic (pack)

Two parts by weight of linolenic acid was homogeneously mixed in usual manner with 1.5 parts by weight of squalene, 0.5 parts by weight of hydrogenated castor oil, 2.0 parts by weight of sodium propionate, 4.0 parts by weight of glyceride, 15.0 parts by weight of polyvinyl alcohol, 10.0 parts by weight of ethanol, and 65.0 parts by weight of purified water to obtain a pack.

Similarly as the product in Example 18, the pack can be advantageously used as a tyrosinase formation suppressing agent in the prevention and treatment of chromatosis.

Example 20

Cosmetic (cream)

Two parts by weight of polyoxyethylene glycol monostearate, 5 parts by weight of glyceryl monostearate SE, 5 parts by weight of propionic acid, 1 part by weight of behenyl alcohol, 2.0 parts by weight of 4,8,12,16-eicosatetraenoic acid, 1 part by weight of liquid paraffine, 10 parts by weight of glyceryl tri (2-ethylhexanoate), and small amounts of antiseptic and flavoring agent were dissolved by heating in usual manner. To the mixture was added 2 parts by weight of normal butyric acid, 5 parts by weight of 1,3-buthylene glycol, and 66 parts by weight of purified water, and the resultant mixture was homogenized to obtain a cream.

Similarly as the product in Example 18, the cream can be advantageously used as a tyrosinase formation suppressing agent in the prevention and treatment of chromatosis.

Effect of the invention

As described in the above, the present inventors found that a substance containing one or more of propionic acid, butyric acid, valeric acid, and their salts and derivatives in combination with or without unsaturated acid(s) exhibited a strong suppressing activity on tyrosinase formation in a pigment cell, but substantially did not inhibit tyrosinase activity, and the present inventors obtained a tyrosinase formation suppressing agent containing the substance as the effective component.

Furthermore, since the present tyrosinase formation suppressing agent exerts a strong melanogenesis suppressing effect and a high efficacy in realizing a pigmentation-lightened skin, the tyrosinase formation suppressing agent can be advantageously used in pharmaceuticals, for example, injection, oral agent, medicine for external application, and bath preparation such as bath salts or bath liquid, and in cosmetics, for example, milky lotion, pack, and cream as a pigmentation-lightening agent in the prevention and treatment of local and systemic chromatosis such as chloasma, freckle, sunburn and addisonism.

The present tyrosinase formation suppressing agent can be used with no side effect because the agent is prepared from organic acids, and their salts and derivatives, and unsaturated fatty acids which are responsible for metabolism and are orally administrable, as well as in view of the effective dose. Furthermore, since thermal- and pH-stabilities of the agent are high, its heat sterilization and long preservation are effected without special care, and the agent can be advantageously produced and used in the field such as pharmaceuticals and cosmetics. Therefore, the present tyrosinase formation suppressing agent is a significant substance in the field.

While there has been described what is at present considered to be the preferred embodiments of the invention, it will be understood that various modifications may be made therein, and it is intended to cover in the appended claims all such modifications as fall within the true spirits and scope of the invention.

**Claims**

1. A pharmaceutical agent to suppress the formation of tyrosinase per se, which comprises one or more members selected from the group consisting of propionic acid, butyric acid and valeric acid, and their salts and derivatives as the effective component.

2. The pharmaceutical agent in accordance with claim 1, wherein said salts of propionic acid, butyric acid and valeric acid are alkali metal salts and alkaline-earth metal salts of propionic acid, butyric acid and valeric acid.

3. The pharmaceutical agent in accordance with claim 1, wherein said derivatives of propionic acid, butyric acid and valeric acid are esters and acid amides of propionic acid, butyric acid and valeric acid.

4. The pharmaceutical agent in accordance with claim 1, wherein said pharmaceutical agent is an injection, tablet, capsule, ointment, lotion or milky lotion.

5. The pharmaceutical agent in accordance with claim 1, wherein said pharmaceutical agent is a cosmetic.

6. The pharmaceutical agent in accordance with claim 5, wherein said cosmetic is an injection, tablet, capsule, ointment, bath salts, lotion, milky lotion, pack or cream.

7. The pharmaceutical agent in accordance with claim 1, wherein a dose of said pharmaceutical agent is in the range of 0.01-1,000 g/day/adult.

8. The pharmaceutical agent in accordance with claim 1, wherein said pharmaceutical agent additionally contains one or more members selected from the group consisting of unsaturated fatty acids, and their salts and derivatives.

9. The pharmaceutical agent in accordance with claim 8, wherein said unsaturated fatty acids are those having 12 to 22 carbon atoms.

10. The pharmaceutical agent in accordance with claim 9, wherein said unsaturated fatty acids having 12 to 22 carbon atoms are lauroleic acid, myristoleic acid, pentadecenoic acid, cis-9-hexadecenoic acid, oleic acid, linoleic acid, linolenic acid, gadoleic acid, arachidonic acid 4,8,12,16-eicosatetraenoic acid, 5,8,11,14,17-eicosapentaenoic acid, erucic acid and clupanodonic acid.

11. The pharmaceutical agent in accordance with claim 8, wherein said salts of unsaturated fatty acids are alkali metal salts and alkaline-earth metal salts of unsaturated fatty acids.

12. The pharmaceutical agent in accordance with claim 8, wherein said derivatives of unsaturated fatty acids are esters and acid amides of unsaturated fatty acids.

13. The pharmaceutical in accordance with claim 8, wherein said pharmaceutical agent is a cosmetic.

14. The pharmaceutical agent in accordance with claim 13, wherein said cosmetic is an injection, tablet, capsule, ointment, bath salts, lotion, milky lotion, pack or cream.

15. The pharmaceutical agent in accordance with claim 8, wherein a dose of said pharmaceutical agent is in the range of 0.01-500 g/day/adult.